# EUROPÄISCHE PATENTSCHRIFT

(11) **EP 1 191 960 B1**
(45) Veröffentlichungstag und Bekanntmachung des Hinweises auf die Patenterteilung: **03.09.2008**
(21) Anmeldenummer: 00947816.5
(22) Anmeldetag: 30.06.2000
(51) Int. Cl.: A61M 1/16, A61K 9/10

(54) **BEHÄLTER MIT BESTANDTEILEN DES SAUREN KONZENTRATS FÜR EINE DIALYSIERFLÜSSIGKEIT UND VERFAHREN ZUR HERSTELLUNG VON SAUREM DIALYSEKONZENTRAT**
CONTAINER WITH COMPONENTS OF AN ACIDIC CONCENTRATE FOR A DIALYSIS FLUID AND METHOD OF PREPARING AN ACIDIC DIALYSIS CONCENTRATE
RECIPIENT AVEC COMPOSES DU CONCENTRE ACIDE D'UN LIQUIDE DE DIALYSE ET METHODE DE PREPARATION DE CE LIQUIDE

(30) Priorität: 06.07.1999 DE 19931077
(43) Veröffentlichungstag der Anmeldung: 03.04.2002
(73) Patentinhaber: Sterisafe GmbH, 14547 Buchholz (DE)
(72) Erfinder: PIPPERT, Manfred, D-61479 Glashütten (DE); HILDMANN, Uwe, D-98708 Gehren (DE); EIGENDORF, Hans-Günther, D-15526 Bad Saarow-Pieskow (DE)
(74) Vertreter: Flosdorff, Jürgen
(86) Internationale Anmeldenummer: PCT/DE2000/002051
(87) Internationale Veröffentlichungsnummer: WO 2001/002036

(56) Entgegenhaltungen:
- EP-A- 0 086 553
- EP-A- 0 456 928
- EP-A- 0 575 970
- DE-C- 4 211 455
- US-A- 4 137 168
- US-A- 4 336 881

## Beschreibung

Die Erfindung bezieht sich auf eine Darreichungsform für die Bestandteile des sauren Dialysekonzentrats, wobei die für eine Dialysebehandlung an einem Patienten erforderliche Quantität an Inhaltsstoffen des sauren Konzentrats in einen Behälter aufgenommen ist. Außerdem betrifft die Erfindung eine Vorrichtung, mit der der Behälterinhalt mit einer vorbestimmten Quantität Wasser vermischt und das fertig aufbereitete saure Dialysekonzentrat der Dialysemaschine zum Herstellen der zur Behandlung vorgesehenen Dialysierflüssigkeit zugeführt wird.

Die bei der Durchführung einer Dialyse an einem Patienten verwendete Dialysierflüssigkeit hat im allgemeinen folgende Bestandteile, deren An- oder Abwesenheit und quantitative Verhältnisse je nach Anwendungsfall unterschiedlich sein können: Natriumchlorid, Natriumhydrogencarbonat, Glukose, Kaliumchlorid, Calziumchlorid, Magnesiumchlorid, Essigsäure, Salzsäure, Natriumacetat, Kalziumglukonat, Ascorbinsäure, Zitronensäure, Hydrate dieser Verbindungen, Wasser geeigneter Qualität für die Dialyse und gegegebenenfalls weitere Inhaltsstoffe.

Bei der allgemein als "Bicarbonat-Hämodialyse" bezeichneten Anwendungsform werden ein sogenanntes "Saures Konzentrat" und ein sogenanntes "Basisches Konzentrat" in definierten Anteilen mit einer vorbestimmten Menge Wasser zur Dialysierflüssigkeit gemischt. Das Basische Konzentrat enthält immer Natriumhydrogencarbonat, gegebenenfalls weitere Bestandteile ausschließlich der Erdalkalisalze und Säuren. Das Saure Konzentrat kann alle notwendigen Bestandteile außer Natriumhydrogencarbonat enthalten.

Dabei bezeichnen die Begriffe "Saures Konzentrat" und "Basisches Konzentrat" nicht unbedingt die Gesamtheit sämtlicher Rohstoffe, die in der schließlich zur Anwendung gelangenden Dialysierflüssigkeit enthalten sind, sondern es können zur Herstellung der Dialysierflüssigkeit auch noch Stoffe hinzugefügt werden, die gemeinsam mit den Dialysekonzentraten und dem Wasser die gebrauchsfertige Dialysierflüssigkeit ergeben.

Seit langem ist es üblich, die Inhaltsstoffe des Dialysekonzentrats in einer Produktionsanlage mit definierten Mengen Wasser zu mischen bzw. in Wasser aufzulösen und als komplett gelöstes Dialysekonzentrat zu einer Dialysestation zu transportieren. Wegen des hohen Wasseranteils in diesen Lösungen ist dies mit einem beträchtlichen Transport- und Lageraufwand verbunden. Deshalb wird seit einiger Zeit die basische Komponente zur Herstellung der Dialysierflüssigkeit -das Natriumhydrogencarbonat- zunehmend als Pulver an das Dialysegerät gebracht und dort unter Hinzufügen von Wasser zum Basischen Konzentrat gelöst.

Es ist auch bereits in geringem Umfang Praxis, die Bestandteile des Sauren Konzentrates zur Herstellung der Dialysierflüssigkeit in Pulverform am Ort der Dialyse zur Verfügung zu stellen, wodurch der Transport- und Lageraufwand verringert ist. Die einzelnen Bestandteile müssen dann vor Ort mit hoher Genauigkeit dosiert werden. Dies erfordert einen erheblichen technischen und personellen Aufwand, Es ist unter den gegebenen Bedingungen einer Dialysestation nicht sicher zu verifizieren, ob die einzelnen Bestandteile tatsächlich in dem vorgegebenen Mischungsverhältnis enthalten sind. Außerdem ist dieses Verfahren aus hygienischen Gründen umstritten. Hieraus ergeben sich hohe Risiken für den Patienten, da aus fehlerhafter Zusammensetzung der Dialysierflüssigkeit gesundheitliche Schäden, im Extremfall mit Todesfolge, resultieren können.

Die EP 0 697 220 A1 schlägt eine flexible medizinische Verpackungseinheit vor, die mit einer einzigen chemischen Verbindung, nämlich einem pulverförmigen Bicarbonat teilweise gefüllt ist. Die Verpackungseinheit hat ein solches Innenvolumen, daß sich das pulverförmige Bicarbonat auch bei völliger Füllung mit Wasser nur teilweise auflöst, wobei dieser Vorgang bis zum Ende der Dialyse mehrere Male so wiederholt wird, dass immer eine gesättigte Lösung von der Dialysemaschine entnommen werden kann.

Die DE 42 11 455 C1 offenbart ein Verfahren und eine Vorrichtung zur Bereitung von Dialysierflüssigkeit aus wässrigen Konzentraten, wobei zur Anpassung der Zusammensetzung der Dialysierflüssigkeit an die individuellen Erfordernisse des Patienten maximale zwei Grundkonzentrate von einheitlich definierter Zusammensetzung und ein den individuellen Erfordernissen entsprechend zusammengesetztes Zusatzkonzentrat getrennt voneinander kontinuierlich zugeführt werden.

EP-A-0 456 928 offenbart eine pastenförmige Dialysatzusammensetzung sowie ein Verfahren zu deren Herstellung, bei dem Natriumchlorid in Pulverform mit einer Partikelgröße von bis zu 105 µm und eine wässrige Lösung weiterer Bestandteile in ein Mischgerät eingegeben und darin vollständig gemischt und gemahlen werden, so dass ein pastenförmiges Produkt entsteht. Der Zweck dieses Verfahrens besteht darin, ein vollständig homogenes pastenförmiges Produkt herzustellen, so dass zur Herstellung von Dialysierflüssigkeit zur Durchführung einer Dialyse eine entsprechende Menge des Produkts entnommen wird, wobei durch die homogene pastöse Form gewährleistet ist, dass jede entnommene Menge dieselbe chemische Zusammensetzung hat. Die Druckschrift erwähnt, dass sich bei einer Partikelgröße über 105 µm des Natriumchlorids keine homogene pastöse Form des Produkts ausbilden lässt, sondern dass eine Suspension entsteht, die ausdrücklich als ungeeignet für Dialysezwecke bezeichnet wird.

EP-A-0 443 324 offenbart ein System zum Vorbereiten einer Flüssigkeit für medizinische Zwecke. Dabei wird wenigstens eine Kartusche, die ein Pulver enthält, in einen Rezirkulationskreislauf eingeschaltet, wodurch das Pulver zur Herstellung der gewünschten Flüssigkeit gelöst wird. In der Druckschrift findet sich der Hinweis, dass mehrere Kartuschen in den Kreislauf eingeschaltet werden können, die ein oder mehr Pulverkonzentrate und möglicherweise auch flüssigkeitsbasiertes Konzentrat enthalten können. Diese allgemeine, nicht näher erläuterte Bemerkung bedeutet aber nicht, dass in ein und dem selben Behälter sowohl pulverförmiges Konzentrat als auch flüssiges Konzentrat vorliegen.

Der vorliegenden Erfindung liegt die Aufgabe zugrunde, Einsparungseffekte bei Transport, Lagerung und Handling im Zusammenhang mit dem Sauren Dialysekonzentrat zu erreichen, wobei die im Dialysekonzentrat enthaltenen wesentlichen Komponenten mit der notwendigen Genauigkeit in ein Behältnis dosiert sein sollen, damit die Sicherheit der Dialysebehandlung nicht eingeschränkt wird. Außerdem soll eine Vorrichtung zum Aufbereiten des Sauren Dialysekonzentrats und ein Verfahren zur Herstellung des Sauren Dialysekonzentrats angegeben werden.

Diese Aufgaben werden erfindungsgemäß durch die Merkmale der Patentansprüche 1, 2 und 3 gelöst.

Vorteilhafte Weiterbildungen der Erfindung sind in den abhängigen Ansprüchen gekennzeichnet.

Die Erfindung sieht vor, daß alle wesentlichen Bestandteile des Sauren Dialysekonzentrats in einem Behältnis als Aufschlämmung vorliegen. Hierdurch werden das Volumen und das Gewicht der zu transportierenden, zu lagernden und zu handhabenden Bestandteile des Sauren Dialysekonzentrats auf ein Drittel bis ein Sechstel gegenüber den herkömmlichen Vorgehensweisen reduziert, wobei die physiologisch notwendige Homogenität des an der Dialysemaschine hergestellten Konzentrats durch die kontrollierte industrielle Fertigung, die die Erfindung kostengünstig ermöglicht, mit der erforderlichen Dosiergenauigkeit der Bestandteile gewährleistet ist. Dies wird ferner durch das erfindungsgemäße Verfahren und die erfindungsgemäße Vorrichtung erreicht.

Die Erfindung schafft die Voraussetzung, die auch aus der herkömmlichen industriellen Konzentratfertigung bekannten Vorteile hinsichtlich Dosiergenauigkeit, Prüfbarkeit bis zur gesetzlich geforderten Rückverfolgbarkeit einzuhalten und dennoch die vorgenannte Volumen- und Massereduktion zu erreichen. Die Erfindung erlaubt ferner, die im Dialysekonzentrat enthaltenen Komponenten mit geringem Masseanteil bei der industriellen Fertigung als Lösung kostengünstig aus einem geprüften größeren Batch mit notwendiger und nachweisbarer Genauigkeit in das Behältnis zu dosieren.

Bei dem Behältnis kann es sich um einen Kunststoffbeutel handeln, aus dem die Aufschlämmung in eine Kartusche eingefüllt wird, die ihrerseits in eine Aufbereitungsvorrichtung einsetzbar ist. Mit großem Vorteil wird aber vorgeschlagen, daß es sich bei dem Behältnis um eine solche Kartusche handelt.

Die Aufschlämmung enthält Natriumchlorid als festen Bestandteil. Der feste Bestandteil ist in einer Lösung der übrigen Bestandteile aufgeschlämmt. Aus dieser Aufschlämmung wird in der weiter unten beschriebenen Vorrichtung an oder in der Dialysemaschine durch Zugabe einer definierten Menge Wasser geeigneter Qualität im Batch-Verfahren ein flüssiges, saures Konzentrat -vergleichbar mit den üblichen Fertigkonzentraten- hergestellt, das mit den bestehenden Dosier- und Sicherheitssystemem marktüblicher Dialysegeräte zu Dialysierflüssigkeit verarbeitet werden kann.

Dabei liegt es im Rahmen der Erfindung, daß die Bestandteile der basischen Komponenten in einem separaten Behälterteil in ungelöster Form enthalten sein können. Diese werden am Dialysegerät so mit Wasser gemischt, daß sie in gelöster Form vorliegen und zur Herstellung von Dialysierflüssigkeit verwendet werden können. Grundsätzlich können auf diese Weise, abgesehen von der benötigten Menge Wasser, alle Bestandteile der Dialysierflüssigkeit mit geringstem Transport- und Lageraufwand an das Dialysegerät gebracht werden.

Die zur Aufnahme der Aufschlämmung verwendete Kartusche kann ein aus Kunststoff oder Glas bestehender formstabiler Körper oder ein flexibler Beutel sein, der nach der Befüllung mit der Aufschlämmung flüssigsdicht und gasdicht verschlossen wird. Dabei ist vorgesehen, daß Zufluß und Abfluß der sogenannten "Sauren Kartusche" so gestaltet sind, daß in der strömenden Flüssigkeit befindliche ungelöste Stoffbestandteile beim Austritt aus der Kartusche zurückgehalten werden.

Die Erfindung betrifft ferner eine Vorrichtung zum Aufbereiten der Aufschlämmung der Kartusche zu Saurem Konzentrat. Die Vorrichtung enthält eine Aufnahmeeinrichtung für die Kartusche, die ein flüssigkeitsdichtes Einfügen in die Zirkulation ermöglicht. Weiterhin gehören ein Batch-Gefäß, das vorzugsweise mit einem Füllstandssensor versehen ist, der nach Erreichen eines vorbestimmten Füllstandes den weiteren Wasserzulauf unterbricht, Leitungen, die Einlaßöffnung und Auslaßöffnung der Kartusche mit einer Einlaßöffnung und einer Auslaßöffnung des Batch-Gefässes verbinden, ein Wasserzulauf und ein Konzentratablauf sowie eine Pumpe zu der Vorrichtung, die das zugeführte Wasser bzw. die Lösung vorzugsweise entgegen der Schwerkraft solange durch das Batch-System zirkulieren läßt, bis sich der feste Bestandteil der Aufschlämmung aufgelöst hat und eine homogene Lösung im System vorliegt. Dies kann von einer Steuereinheit mittels Sensoren überwacht oder zeitgesteuert werden und in einem Signal zur Öffnung des Auslaßventils münden.

Diese Vorrichtung kann in ein Dialysegerät integriert sein oder als eine Art Zusatzgerät an dem Dialysegerät angeordnet werden.

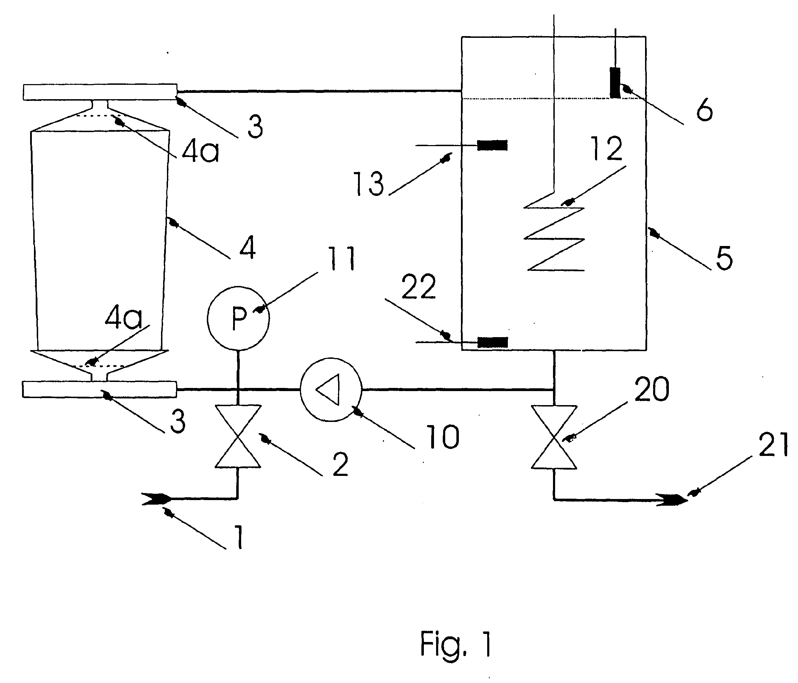

Weitere Einzelheiten der Vorrichtung zur Aufbereitung der Aufschlämmung ergeben sich aus der nachfolgenden Beschreibung einer bevorzugten Ausführungsform sowie anhand der beigefügten Zeichnung. Diese zeigt auf rein schematische Weise eine derartige Vorrichtung.

Über einen Anschluß 1 wird Wasser geeigneter Qualität in die Vorrichtung eingelassen. Bevorzugt handelt es sich um Wasser aus dem Dialysegerät, das entgast und temperiert ist. Wenn derartiges Wasser nicht zur Verfügung steht, kann anderweitig verfügbares Wasser geeigneter Qualität verwendet werden, das in der Vorrichtung vorzugsweise erwärmt werden kann.

Nach Anschluß einer Sauren Kartusche 4 an die beiden Anschlüsse 3 wird ein Zulaufventil 2 geöffnet, so daß Wasser vorzugsweise von unten in die Saure Kartusche eintritt. Die beiden Anschlüsse 3 sind vorzugsweise an gegenüberliegenden Seiten der Saure Kartusche angebracht, können aber auch nebeneinander auf der gleichen Seite oder beliebig anders lokalisiert sein. An Zu- und Ablauf der Sauren Kartusche sind jeweils Filter 4a vorgesehen, die das Austreten ungelöster Bestandteile aus der Kartusche verhindern. Nach Einströmen von Wasser in die Saure Kartusche fließt die entstehende Lösung in ein Batch-Gefäß 5. Ein Füllstandsensor 6 beendet den Wasserzulauf. Der Füllstandsensor ermöglicht die Festlegung auf unterschiedliche, genau definierte Verdünnungsverhältnisse, wobei das gesamte Batch-Volumen der Summe der Volumina von Batch-Gefäß 5, Saure Kartusche 4 und dem zugehörigen Schlauchsystem entspricht. Ersatzweise kann bei einer anderen Version das Volumen der Sauren Kartusche durch Entleeren eliminiert werden.

Der Füllstandsensor 6 ist vorzugsweise als Leitfähigkeitssensor ausgebildet und ermöglicht so eine zusätzliche Kontrolle der Lösung der Feststoffe.

Nach Füllung des gesamten Batch-Volumens wird eine Pumpe 10 gestartet, die die Flüssigkeit in dem System zirkulieren läßt und damit die vollständige Auflösung der Feststoffe beschleunigt und zeitabhängig Homogenität bewirkt.

Als Sicherheitsüberwachung ist ein Drucksensor 11 vorgesehen. Die Vorrichtung enthält ferner bevorzugt eine Heizung 12, um das Lösen der Feststoffe zu beschleunigen, und einen Temperatursensor 13. Der Temperatursensor 13 dient einerseits der Heizungsregelung und kann andererseits in Verbindung mit der Leitfähigkeitsmeßzelle 6 mit der notwendigen Genauigkeit die Vollständigkeit der Auflösung der Feststoffe nachweisen.

Nach dem vollständigen Lösen der Feststoffe und homogener Verteilung der Bestandteile ist ein verwendungsfähiges Saures Dialysekonzentrat entstanden. Es wird nun ein Auslaßventil 20 geöffnet, um das Dialysekonzentrat durch die Auslaßleitung 21 zum Dialysegerät weiterzuleiten.

Das Auslaßventil 20 kann vor oder nach der Pumpe angeordnet sein.

Außerdem kann die Vorrichtung einen Niveausensor 22 enthalten, der einen minimalen Flüssigkeitsstand meldet.

## Patentansprüche

1. Verfahren zur Herstellung von Saurem Dialysekonzentrat mit folgenden Schritten:
a) Eine wässrige Lösung aller Bestandteile des Sauren Dialysekonzentrates mit Ausnahme von NaCl wird hergestellt;
b) Die wässrige Lösung und NaCl in Pulver oder Granulatform werden in ein Behältnis gefüllt, so dass die Bestandteile als Aufschlämmung in dem Behältnis vorliegen;
c) Das Behältnis wird in eine Aufnahmeeinrichtung einer Vorrichtung zum Aufbereiten des Dialysekonzentrats eingesetzt und
d) Wasser wird durch das Behältnis zirkuliert, bis die festen Bestandteile aufgelöst sind.

2. Behältnis mit allen Bestandteilen des Sauren Dialysekonzentrats zur Durchführung der Schritte c) und d) des Verfahrens nach Anspruch 1, wobei NaCl in Pulver- oder Granulatform und die übrigen Bestandteile in einem in Wasser gelösten Zustand in das Behältnis dosiert sind, so dass die Bestandteile als Aufschlämmung in dem Behältnis vorliegen.

3. Vorrichtung zur Durchführung des Verfahrens nach Anspruch 1,
**gekennzeichnet durch**
eine Aufnahmeeinrichtung mit einem lösbar eingesetzten Behältnis nach Anspruch 2, ein Batch-Gefäß (5), verbindende Leitungen, einen Wasserzulauf (1) und einen Ablauf (21) sowie eine Pumpe (11) zur Zirkulation des zugeführten Wassers bzw. des sich bildenden Konzentrats, um die festen Bestandteile vollständig aufzulösen und homogen im Zirkulationssystem zu verteilen.

4. Vorrichtung nach Anspruch 3,
**dadurch gekennzeichnet,**
**daß** die Vorrichtung in ein Dialysegerät integriert ist.

5. Vorrichtung nach Anspruch 3,
**dadurch gekennzeichnet,**
**daß** die Vorrichtung als Zusatzgerät mit einem Dialysegerät verbindbar ist.

6. Vorrichtung nach einem der Ansprüche 3 bis 5,
**dadurch gekennzeichnet,**
**daß** die Vorrichtung eine Füllstandsregeleinrichtung enthält, die eine exakte Dosierung von Wasser geeigneter Qualität ermöglicht.

7. Vorrichtung nach einem der Ansprüche 3 bis 6,
**dadurch gekennzeichnet,**
**daß** die Vorrichtung eine Flüssigkeitsdosiereinrichtung enthält, die eine exakte Dosierung von Wasser geeigneter Qualität ermöglicht.

8. Vorrichtung nach einem der Ansprüche 3 bis 7,
**dadurch gekennzeichnet,**
**daß** eine Reinigungs-/Desinfektionseinrichtung zur Reinigung und Desinfektion der gesamten Vorrichtung vorgesehen ist.

9. Vorrichtung nach einem der Ansprüche 3 bis 8,
**dadurch gekennzeichnet,**
**daß** eine Steuereinrichtung alle Regel- und Steuerfunktionen durchführt und die Sicherheit der Vorrichtung gewährleistet.

## Claims

1. Method of producing acidic dialysis concentrate with the following steps:
a) An aqueous solution of all constituents of the acidic dialysis concentrate with the exception of NaCl is produced;
b) The aqueous solution and NaCl in powdered or granular form are introduced into a container so that the constituents are present in the container as a suspension;
c) The container is introduced into a receiving means of a device for preparing the dialysis concentrate, and
d) Water is circulated through the container until the solid constituents are dissolved.

2. Container with all constituents of the acidic dialysis concentrate for carrying out the steps c) and d) of the method as claimed in Claim 1, wherein NaCl in powdered or granular form and the remaining constituents are dissolved in water and are metered into the container, so that the constituents are present in the container as a suspension.

3. Apparatus for carrying out the method as claimed in Claim 1, **characterised by** a detachably inserted container as claimed in Claim 2, a batch vessel (5), connecting lines, a water inlet (1) and an outlet (21) as well as a pump (11) for circulating the supplied water and the developing concentrate in order to completely dissolve the solid constituents and to distribute them homogeneously in the circulation system.

4. Apparatus as claimed in Claim 3, **characterised in that** the apparatus is integrated into a dialyser.

5. Apparatus as claimed in Claim 3, **characterised in that** the apparatus can be connected as an accessory device to a dialyser.

6. Apparatus as claimed in any one of Claims 3 to 5, **characterised in that** the apparatus includes a filling level control means which enables exact metering of water of a suitable quality.

7. Apparatus as claimed in any one of Claims 3 to 6, **characterised in that** the apparatus includes a liquid metering means which enables exact metering of water of a suitable quality.

8. Apparatus as claimed in any one of Claims 3 to 7, **characterised in that** a cleaning and disinfecting means is provided for cleaning and disinfecting the entire apparatus.

9. Apparatus as claimed in any one of Claims 3 to 8, **characterised in that** a control means carries out all regulating and control functions and ensures the reliability of the apparatus.

## Revendications

1. Procédé de réalisation d'un concentré de dialyse acide, comportant les étapes suivantes :
a) réalisation d'une solution aqueuse de tous les constituants du concentré de dialyse acide, à l'exception du NaCl ;
b) introduction de la solution aqueuse et du NaCl en poudre ou sous forme de granulés dans un récipient, de telle sorte que les constituants sont présents sous forme de suspension dans le récipient ;
c) introduction du récipient dans un dispositif de réception d'un dispositif de préparation du concentré de dialyse ; et
d) circulation de l'eau à travers le récipient jusqu'à ce que les constituants solides soient dissous.

2. Récipient contenant tous les constituants du concentré de dialyse acide, destiné à la mise en oeuvre des étapes c) et d) du procédé selon la revendication 1, dans lequel le NaCl en poudre ou en forme de granulés et les autres constituants à l'état dissous dans l'eau sont dosés dans le récipient, de telle sorte que les constituants sont présents sous forme de suspension dans le récipient.

3. Dispositif destiné à la mise en oeuvre du procédé selon la revendication 1, **caractérisé par** un dispositif de réception comportant un récipient selon la revendication 2, mis en place de manière amovible, un récipient de traitement par lots (5), des conduites de raccordement, une admission d'eau (1) et une évacuation (21), ainsi qu'une pompe (11) pour la circulation de l'eau acheminée ou du concentré se formant, afin de dissoudre complètement les constituants solides et de les répartir de manière homogène dans le système de circulation.

4. Dispositif selon la revendication 3, **caractérisé en ce que** le dispositif est intégré dans un appareil de dialyse.

5. Dispositif selon la revendication 3, **caractérisé en ce que** le dispositif, en tant qu'appareil supplémentaire, peut être relié à un appareil de dialyse.

6. Dispositif selon l'une quelconque des revendications 3 à 5, **caractérisé en ce que** le dispositif comporte un dispositif de réglage du niveau de remplissage, qui permet un dosage exact de l'eau de qualité appropriée.

7. Dispositif selon l'une quelconque des revendications 3 à 6, **caractérisé en ce que** le dispositif comporte un dispositif de dosage du liquide, qui permet un dosage exact de l'eau de qualité appropriée.

8. Dispositif selon l'une quelconque des revendications 3 à 7, **caractérisé en ce qu'**il est prévu un dispositif de nettoyage et de désinfection, destiné à nettoyer et à désinfecter l'ensemble du dispositif.

9. Dispositif selon l'une quelconque des revendications 3 à 8, **caractérisé en ce qu'**un dispositif de commande exécute toutes les fonctions de réglage et de commande et garantit la sécurité du dispositif.
